Europäisches Patentamt

(19) European Patent Office

Office européen des brevets

(11) Numéro de publication: **0 083 261**
**B1**

(12) **FASCICULE DE BREVET EUROPEEN**

(45) Date de publication du fascicule du brevet: **20.04.88**

(51) Int. Cl.⁴: **A 61 K 31/70** // A61K9/08

(21) Numéro de dépôt: **82402232.1**

(22) Date de dépôt: **07.12.82**

(54) **Composition thérapeutique, à base d'inosine monophosphaté, pour le traitement des troubles d'accommodation de l'oeil.**

(30) Priorité: **30.12.81 FR 8124476**

(43) Date de publication de la demande:
**06.07.83 Bulletin 83/27**

(45) Mention de la délivrance du brevet:
**20.04.88 Bulletin 88/16**

(84) Etats contractants désignés:
**AT BE CH DE GB IT LI NL**

(56) Documents cités:
**CHEMICAL ABSTRACTS, vol. 80, no. 19, 13 mai 1974, page 39, no. 104049r, Columbus,Ohio (USA); S.C. TRACHTENBERG et al.: "Effect of inosine on blood pressure in the rat".**
**CHEMICAL ABSTRACTS, vol. 76, no. 7, 14 février 1972, page 21, no. 30642x, Columbus,Ohio (USA); McDONALD, THOMAS O. et al.: "Polyinosinic acid-polycytidylic acid in ophthalmology. III. Intraocular response after intravenous, intraocular, and topical ocular administration in rabbits".**

(73) Titulaire: **Laboratoires P.O.S. Société Anonyme dite:**
**21 rte de Lapoutroie**
**F-68240 Kaysersberg (FR)**

(72) Inventeur: **Andermann, Guy**
**5, rue des Jonquilles**
**F-68000 Colmar (FR)**
Inventeur: **Andermann, Claudine**
**5, rue des Jonquilles**
**F-68000 Colmar (FR)**

(74) Mandataire: **Cuer, André**
**CABINET CUER 30, rue de Léningrad**
**F-75008 Paris (FR)**

(56) References cited:
**CURRENT EYE RESEARCH, vol. 1, no. 7, 1981, pages 375-380, IRL Press Limited, Londres (GB); S.J. KOPP et al.: "Analysis of intact rat lens metabolites by P-31 NMR spectroscopy".**

Courier Press, Leamington Spa, England.

**0 083 261**

(56) References cited:

**THE MERCK INDEX, AN ENCYCLOPEDIA OF CHEMICALS AND DRUGS, 1976, 9th edition, New York (USA); page 137, ref. nr. 1059: "Benzalkonium Chloride", page 265, nr. 2060: "Chlorhexidine", page 1202, ref. nr. 9046: "Thimerosal".**

**Dictionnaire VIDAL (1982) p. 216 "CATACOLPOS"**

## Description

La présente invention a trait au domaine des compositions pharmaceutiques destinées aux traitements oculaires et concerne tout spécialement une composition thérapeutique apte à traiter les toubles de l'accommodation de l'oeil humain tels que strabismes, presbyties, hétérophories.

On a déjà préconisé pour des traitements de ce type des associations médicamenteuses à éléments biochimiques dont, en particulier, une combinaison bien spécifique pour le traitement local de la cataracte renfermant les ingrédients : nicotinamide, inosine monophosphate, succinate de sodium, L-aspartate de magnésium et, éventuellement, glycérophosphate de sodium.

Il a maintenant été trouvé de façon surprenante qu'un seul de ces produits, l'inosine monophosphate avait une·action bénéfique dans le traitement de nombreux troubles de la vision et qu'un collyre à base de ce produit actif corrigeait de façon notable le rapport AC/A d'un oeil malade pendant plusieurs mois après le traitement (A = Accommodation, C = Convergence).

L'inosine monophosphate est un nucléotide bien connu en soi, utilisé par exemple comme agent potentialisateur de certains arômes alimentaires, notamment en association avec le monophosphate de guanosine. Par ailleurs, plusieurs auteurs ont signalé que l'inosine elle-même, utilisée par voie intramusculaire ou intraveineuse, était douée d'activité analectique cardiaque et pouvait être employée dans le traitement de l'insuffisance cardiaque chez l'homme. Toutefois, à la connaissance de la Demanderesse, on n'a pas décrit à ce jout d'application thérapeutique spécifique de l'inosine monophosphate.

L'invention a donc pour objet une composition thérapeutique, à base de monophosphate d'inosine ou d'un sel sodique pharmaceutiquement acceptable de ce produit et destinée au traitement des troubles d'accommodation de l'oeil, caractérisée en ce qu'elle présente sous la forme d'un collyre en solution aqueuse saline tamponnée contenant 0,01 à 0,2% de sel sodique de monophosphate d'inosine par rapport au poids total de collyre, à condition que ladite composition ne contienne pas la nicotinamide, le succinate de sodium, le glycérophosphate de sodium et le L-aspartate de magnésium.

Un tel collyre ne présente pas les inconvénients connus de courte durée d'action, intolérance locale, toxicité systémique, inhérents à l'emploi des collyres à base de substance myotiques comme par exemple l'iodure de phospholine, le difluorophate ou encore l'ésérine.

Bien que la gamme physiologique normale de pH de l'oeil humain soit de 7,3 à 7,4, les solutions de traitement des yeux ou collyres peuvent présenter, selon la stabilité des divers constituants, un pH de 3 à 9, de préférence de 5,5 à 8,0. Pour que le collyre de l'invention reste bien stable, il est préférable de maintenir le pH entre 6,2 et 7,5.

On peut ajouter au collyre n'importe quel système tampon pharmaceutiquement acceptable, pour maintenir le pH désiré. Parmi les tampons utilisables on peut citer : le borate de sodium, l'acide borique, le monohydrogénophosphate disodique, le dihydrogénophosphate sodique, un tampon citrate composé de citrate de sodium et d'acide citrique ou un tampon acétate composé d'acide acétique et d'acétate de sodium, ainsi que les associations de tampons phosphate et citrate.

Le salinité de la solution préparée est de préférence isotonique, ce qui équivaut à 0,9% de chlorure de sodium, mais elle peut varier de 0,8 à 1,0%, concentration qui est raisonnablement, isotonique dans une composition. On peut utiliser tout sel pharmacologiquement acceptable pour maintenir l'isotonie désirée de la solution obtenue. Etant donné que les nécessités isotoniques peuvent varier d'un patient à l'autre, on peut réaliser l'isotonie désirée pour tout patient en augmentant ou en diminuant la quantité de sel contenue dans la solution. On peut aussi, au lieu d'un sel, ajouter des corps hydrosolubles non ioniques, pharmacologiquement acceptables, pour maintenir ou réaliser l'isotonie désirée. Parmi ces corps non ioniques on peut citer à titre illustratif le polyéthylèneglycol et le polypropylèneglycol.

Il a en outre été trouvé, de façon inattendue, que lorsque l'inosine monophosphate était utilisé seul et non en association avec d'autres composés comme par exemple la nicotinamide, la succinate de sodium, le glycérophosphate de sodium, le L-aspartate de magnésium ou le chlorure de sodium, la préparation était beaucoup plus stable, aussi bien du point de vue bactériologique, que du point de vue physico-chimique. Cette amélioration évite la contamination bactérienne et la transformation de l'inosine monophosphate en hypoxanthine. Par ailleurs, les allergies de contact à certaines substances sont évitées.

Les solutions aqueuses convenant à l'administration topique dans l'oeil peuvent contenir environ 0,01 et 0,2% en poids d'un sel de sodium de l'inosine monophosphate approprié. Il est recommandé de les appliquer à l'oeil à raison de 1 à 4 fois par jour pendant une durée de traitement d'au moins un mois.

L'invention sera mieux comprise par la description ci-dessous relative d'une part à une série d'exemples de collyres aptes à convenir aux buts recherchés et, d'autre part, aux résultats de travaux cliniques réalisés sur de telles compositions à base de monophosphate d'inosine.

A) Exemples de compositions selon l'invention On a réalisé ci-dessous des solutions aqueuses de collyre appropriées à l'administration topique:

Exemple 1

| | |
|---|---|
| Inosine monophosphate, sel disodique | 0,1 g |
| Chlorure de sodium | 0,85 g |
| Edétate disodique | 0,02 g |

| | |
|---|---|
| Tampon phosphate (à pH 6,0) | 0,1 g |
| Chlorure de benzalkonium | 0,005 g |
| Eau | q.s.p. 100 ml |

### Exemple 2

| | |
|---|---|
| Inosine monophosphate, sel disodique | 0,1 g |
| Chlorure de sodium | 0,85 g |
| Edétate disodique | 0,02 g |
| Hydroxyéthylcellulose | 0,3 g |
| Alcool polyvinylique | 0,1 g |
| Tampon phosphate (à pH 6,0) | 0,1 g |
| Chlorure de benzalkonium | 0,005 g |
| Eau | q.s.p. 100 ml |

### Exemple 3

| | |
|---|---|
| Inosine monophosphate, sel disodiqué | 0,1 g |
| Chlorure de sodium | 0,85 g |
| Edétate disodique | 0,02 g |
| Tampon phosphate (à pH 6,0) | 0,1 g |
| Thimérosal | 0,010 g |
| Eau | q.s.p. 100 ml |

### Exemple 4

Analogue à l'exemple II, si ce n'est que l'on remplace le chlorure de benzalkonium (0,005 g) par le thimérosal (0,010 g).

### Exemples 5 à 8

Analogues aux exemples 1 à 4, respectivement, si ce n'est que l'on remplace le tampon phosphate par un tampon borate (à pH 6,0).

### Exemple 9

| | |
|---|---|
| Inosine monophosphate, sel disodique | 0,05 g |
| Chlorure de sodium | 0,85 g |

| | |
|---|---|
| Edétate disodique | 0,02 g |
| Tampon acétate (à pH 6,0) | 0,1 g |
| Chlorure de benzalkonium | 0,005 g |
| Eau | q.s.p. 100 ml |

### Exemple 10

| | |
|---|---|
| Inosine monophosphate, sel disodique | 0,1 g |
| Digluconate de chlorhexidine | 0,005 g |
| Chlorure de sodium | 0,9 g |
| Eau purifiée | q.s.p. 100 ml |

Ainsi, comme on peut le voir, des compositions selon l'invention peuvent contenir des agents stérilisants, antiseptiques et/ou conservateurs connus comme par exemple le chlorure de benzalkonium, le thimérosal, le gluconate de chlorhexidine.

B) Expérimentations cliniques

De nombreuses expérimentations sur malades présentant des troubles d'accommodation de l'oeil ont été entrepris et peuvent être résumés comme suit (sur la base de collyres aqueux correspondant aux exemples ci-dessus).

bl) troubles de l'équilibre oculo-moteur

On a étudié l'action d'un collyre du type de l'exemple n° 10 ci-dessus sur 45 cas répartis comme suite : 7 cas normaux, 7 cas asthénopiques, 8 sujets hétérophoriques, 8 sujets presbytes et 14 sujets strabiques.

Afin de vérifier objectivement l'action du collyre à l'inosine monophosphate, on a fait une mesure du rapport AC/A. Dans tous les cas, les instillations du collyre à l'IMP ont été biquotidiennes pendant 10 à 15 jours.

Il résulte de ces travaux que (en pourcentage de cas traités):
— 82,0% des patients voient une modification favorable du rapport AC/A
— 97,5% des sujets éprouvent une amélioration subjective
— 2,5% des patients ne présentent pas d'amélioration thérapeutique ou subjective.

Cette étude a indiscutablement prouvé l'action bénéfique du collyre sur le rapport qui lie accommodation et convergence. Etant donné la syncinésie accommodation-convergence, la facilité d'accommodation réduit la convergence associée; le rapport élevé s'est ainsi trouvé normalisé dans les asthénopies, les hétérophories de type insuffisance de convergence, ainsi que dans les ésotropies accommodatives. Dans d'autres cas (presbyties, exophories), le rapport abaissé s'est normalisé, là encore, semble-t-il, par la facilité d'accommodation.

Globalement, on peut conclure que l'action pharmacodynamique est comparable à celle des myotiques, mais sans en présenter les inconvénients.

b2) troubles de la vision binoculaire

On a étudié l'action du collyre selon l'exemple n° 10 précité sur 40 patients atteints de strabisymes (10 cdas) presbytie (8 cas), hétérophorie (8), asthénopie (7), affections oculaires diverses (7).

La valeur du rapport AC/A a été déterminée avec l'acamètre. Tous les patients ont reçu 2 instillations par jour pendant 15 jours, puis un jour sur deux, un tel traitement ayant été prolongé chez les strabiques.

Les résultats de ces travaux ont montré que:
— 33 malades, soit 82,5%, ont vu une modification favorable du rapport AC/A
— 7 malades, soit 17,5%, ne présentaient pas de modifications du rapport AC/A mais celui-ci a été maintenu dans tous les cas dans des valeurs normales ou proches des normes. Par ailleurs, ces malades présentaient tous une amélioration portant sur leur courbe d'accommodaation et de convergence ou leur asthénopie.

b3) troubles de la fusion chez les astigmates

On a expérimenté un collyre au monophosphate d'inosine selon l'invention chez 95 patients en les traitant en double aveugle et en leur faisant subir des séries d'examen dont : mesure de l'acuité visuelle, skiascopie, ophtalmométrie, mesure de la fusion (règle de Berens), mesure du rapport AC/A (à l'aide de l'acamètre).

Les patients présentaient une amplitude de fusion abaissée, un astigmatisme de 0,25 à 4 dioptries et un rapport AC/A fortement perturbé. Ils ont été classé en deux groupes, le premier étant traité au collyre selon l'invention alors que le second était traité en double aveugle tout d'abord avec un collyre placébo puis avec le collyre au monophosphate d'inosine.

On a obtenu les résultats suivants:
— Pour le groupe I : 88,2% de très bons résultats fonctionnels; 97,5% d'amélioration sur la fusion et 85,8% de rapport normal pour AC/A
— Pour le groupe II: des résultats inchangés pour les trois types de critères précités mais, après traitement au collyre de l'invention, une notable amélioration de ces critères chez tous les sujets.

On a pu conclure à l'intérêt soutenu d'un tel collyre dans le traitement des troubles de la fusion chez l'astigmate et la normalisation de la vision binoculaire, en évitant ainsi le port de verres correcteurs.

b4) utilisation en orthoptique

L'étude a porté ici sur 72 sujets agés de 2 à 20 ans qui présentaient du strabisme soit par insuffisance de convergence ou ésotropie soit par ésotropie résiduelle post-opératoire; et l'on a recherché et noté systématiquement — par des examens ophtalomologiques et orthoptiques avant, pendant et après traitement — les valeurs des angles objectifs (mesurées au synoptophore) et des angles subjectifs de façon à pouvoir établir les correspondances rétiniennes normales ou anormales.

Les enfants ou adolescents ont été séparés en deux groupes dont : le groupe I contenant 55 sujets ayant reçu des instillations de collyre selon l'invention à raison de 1 goutte deux à trois fois par jour dans les deux yeux (périodes variables 15 jours à 12 mois ou plus); le groupe II composé d'une part : de 17 sujets n'ayant jamais reçu d'instillation de collyre et, d'autre part, de 32 sujets du groupe I et considérés comme témoins pour la période précédent la prescription de collyre selon l'invention. On a pu ainsi effectuer 104 observations sur 72 patients dont on notera que la plupart étaient déjà traités depuis des mois par corrections optique et que certains avaient essuyé des échecs aux méthodes classiques avec d'autres collyres.

On a tout d'abord noté au cours des traitements que les instillations de collyre à l'inosine monophosphate étaient bien tolérées et ne provoquaient ni irritation ni atteintes de la cornée. Il est par ailleurs intéressant de noter qu'il n'a pas été constaté de myosis, comme cela est le cas lors du traitement connu par des collyres myotiques qui provoquent une gêne visuelle importante.

Pour noter les résultats obtenus on a établi une classification du type:

très bon résultant lorsque l'angle disparaissait ou diminuait fortement

résultat moyen quand l'angle diminuait faiblement

résultat nul quand l'angle conservait sensiblement sa valeur de départ

On a pu ainsi constater que chez les patients traités au collyre selon l'invention on obtenait près de 50% de très bons résultats et plus de 30% de résultats moyens et l'on a pu conclure que le collyre à l'inosine monophosphate avait une action indéniable sur les angles chez les strabiques et sur les angles résiduels chez les strabiques opérés pour cette déformation.

**Revendications**

1. Composition thérapeutique, à base de monophosphate d'inosine ou d'un sel sodique pharmaceutiquement acceptable de ce produit et destinée au traitement des troubles d'accommodation de l'oeil, caractérisée en ce qu'elle présente sous la forme d'un collyre en solution aqueuse saline tamponnée contenant 0,01 à 0,2% de sel sodique de monophosphate d'inosine par rapport au poids total de collyre, à condition que ladite composition ne contienne pas la nicotinamide, le succinate de sodium, le glycérophosphate de sodium et le L-aspartate de magnésium.

2. Composition selon la revendication 1, caractérisée en ce qu'elle contient en outre des agents stérilisants, antiseptiques et/ou conservateurs du groupe constitué par : chlorure de benzalkonium, thimérosal, gluconate de chlorhexidine.

**Patentansprüche**

1. Therapeutische Zusammensetzung auf Basis von Inosin-Monophosphat oder einem pharmazeutisch akzeptablen Natriumsalz dieser Verbindung und bestimmt für die Behandlung von Akkomodationsstörungen des Auges, dadurch gekennzeichnet, daß diese in Form von Augentropften als salzige, gepufferte, wässrige Lösung vorliegt, die 0,01 bis 0,2% Natriumsalz des Inosin-Monophosphats, bezogen auf das Gesamtgewicht der Augentropfen, enthält, wobei die besagte Zusammensetzung Nicotinamid, Natriumsuccinat, Natriumglycerophosphat und Magnesium-L-aspertat nicht enthält.

2. Zusammensetzung nach Anspruch 1, dadurch gekennzeichnet, daß diese darüberhinaus sterilisierende antiseptische und/oder konservierende Mittel einer Gruppe enthält, die gebildet wird aus: Benzalkoniumchlorid, Thiomersal, Chlorhexidin-gluconat.

**Claims**

1. A therapeutic composition, based on inosine monophosphate or a pharmaceutically acceptable sodium salt thereof and intended for the treatment of troubles in accommodation to the human eye, characterized by the fact it is under the form of an eyewash in an isotonic buffered saline solution containing 0,01 to 0,2% of sodium salt of inosine monophosphate in relation to the total weight of the eyewash, with the proviso that said composition does not contain nicotinamid, sodium succinate, sodium glycerophosphate and magnesium L-aspartate.

2. Composition according the claim 1, wherein comprising in addition sterilizing, antiseptic and/or preservative agents of the group consisting of : benzalkonium chloride, thimerosal, chlorohexidine gluconate.